# EUROPEAN PATENT APPLICATION

(11) **EP 1 086 672 A2**
(43) Date of publication of application: **28.03.2001**
(21) Application number: 00305532.4
(22) Date of filing: 30.06.2000
(51) Int. Cl.: A61F 5/01

(54) **Orthopaedic brace having a range of motion hinge with radially actuated stops**

(30) Priority: 25.09.1999 US 156126 P
(71) Applicant: DEPUY ORTHOPAEDICS, INC., Warsaw, Indiana 46581 (US)
(72) Inventor: Telles, Jeffrey Lee, Apartment 42 Tracy CA 95376 (US); Crippen, Ryan W, Stockton CA 95203 (US); Kazmierczak, Andy, Tracy CA 95376 (US); Finkes, James Gregory, Patterson CA 95363 (US)
(74) Representative: Belcher, Simon James

(57) **Abstract**

A range of motion (ROM) hinge assembly is provided for an orthopaedic brace. The ROM hinge provides for the selective adjustment of both a flexion angle or a range of motion and an extension angle or range of motion about an axis of the hinge assembly which typically corresponds to a joint axis of a user. The adjustment of the flexion and extension angles is accomplished by radially actuated stops. The stops (one for flexion and one for extension) are positively biassed into one of a plurality of selective positions (one set for flexion and another set for extension) and disengagable by the user to set the respective range of motion of the struts.

## Description

The present application relates to orthopaedic joint braces and, more particularly, to orthopaedic joint braces for use in stabilizing a joint after injury or invasive surgery. Most particularly, the present invention relates to a low-profile range of motion hinge for such braces.

In order to ensure the proper healing of a human joint after an injury or invasive surgery, it is often desirable to limit the pivotal motion of the human joint to a predetermined angular range between full extension and full flexion. The pivotal motion may be limited by a range of motion hinge disposed between an upper strut and a lower strut.

Orthopaedic braces of this general type, including information relating to range of motion braces, and how and why such equipment is used are disclosed in US-4489718, US-4817588, US-4982732, US-5460599, US-5672152, US-5814000 and US-5827208.

It is well known that the range of motion braces described in the aforementioned patents suffer various problems, shortcomings and disadvantages. Most notably, the range of motion hinges tend to be bulky and difficult to operate, manipulate and adjust without the use of tools,

The present invention provides a range of motion hinge for an orthopaedic brace. The range of motion hinge can be set selectively in a plurality of positions corresponding to a plurality of ranges of motion for both flexion and extension of a joint of a user through radially actuated stops.

In one form, the present invention provides an orthopaedic brace that includes a first strut, a second strut, and a hinge assembly disposed between the first strut and the second strut. The hinge assembly is configured to provide movement of one of the first and second struts about a pivot axis and including a radially actuated stop assembly configured to restrict the movement of the first and second struts to a selected range of motion.

In another form, the present invention provides an orthopaedic brace that includes an upper strut, a lower strut, a radially actuated stop assembly, and a hinge assembly disposed between the upper strut and the lower strut. The hinge assembly is configured to provide movement of the upper and lower struts about a pivot axis corresponding to a joint axis of a user. The hinge assembly includes a plurality of notches corresponding to a plurality of ranges of motion and configured to coact with the radially actuated stop assembly to restrict the movement of the upper and lower struts to a selective one of the plurality of ranges of motion.

In yet another form, the present invention provides an orthopaedic brace that includes an upper strut, a lower strut, a radially actuated extension stop assembly, a radially actuated flexion stop assembly, and a hinge assembly disposed between the upper and lower struts. The hinge assembly is configured for movement of the upper and lower struts about a pivot axis corresponding to a joint axis of a user. The hinge assembly further includes a pair of opposing base plates coupled to one of the upper and lower struts and pivotally retaining the other of the upper and lower struts between them. The pair of opposing base plates each has an extension slot with a plurality of extension notches and a flexion slot with a plurality of flexion notches. The extension slots retain the radially actuated extension stop assembly for translation therein to selectively engage one of the plurality of pairs of extension notches to restrict movement of the upper and lower struts to a selected range of extension motion. The flexion slots retain the radially actuated flexion stop assembly for translation therein to selectively engage one of the plurality of pairs of flexion notches to restrict movement of the upper and lower struts to a selected range of flexion motion.

Accordingly, the present invention improves upon the prior art by providing a low-profile, range of motion hinge that is easily adjustable by the wearer. More specifically, the improved range of motion hinge may be adjusted without the use of tools, and it is smaller in diameter, is lighter in weight, and is lower in profile in comparison to the prior art. This cost-effective range of motion hinge allows the selective restriction of range of motion in both extension and flexion through radially actuated stops in an enclosed system of stop ways and stop notches.

The present invention will now be described by way of example with reference to the accompanying drawings, in which:
FIG. 1 is a side perspective view of an adjustable, motion-restraining knee brace containing an improved range of motion hinge assembly that embodies principles of the present invention showing the brace operatively connected to a human leg;
FIGS. 2A and 2B are side elevational views depicting a first embodiment of the range of motion hinge of FIG. 1 showing an arbitrary degree of flexion in FIG. 2A and a full degree of extension in FIG 2B;

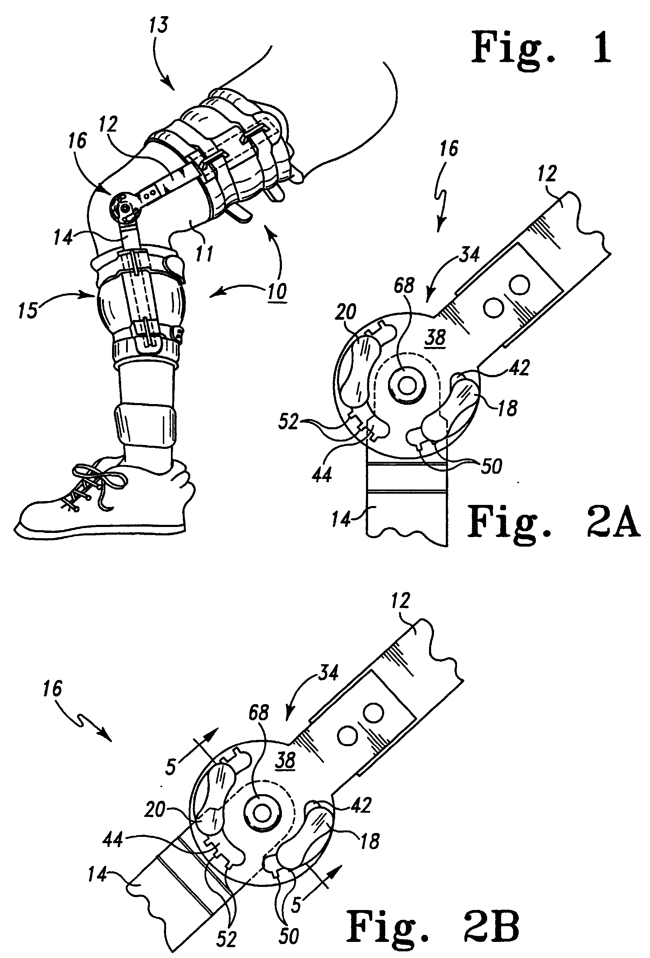

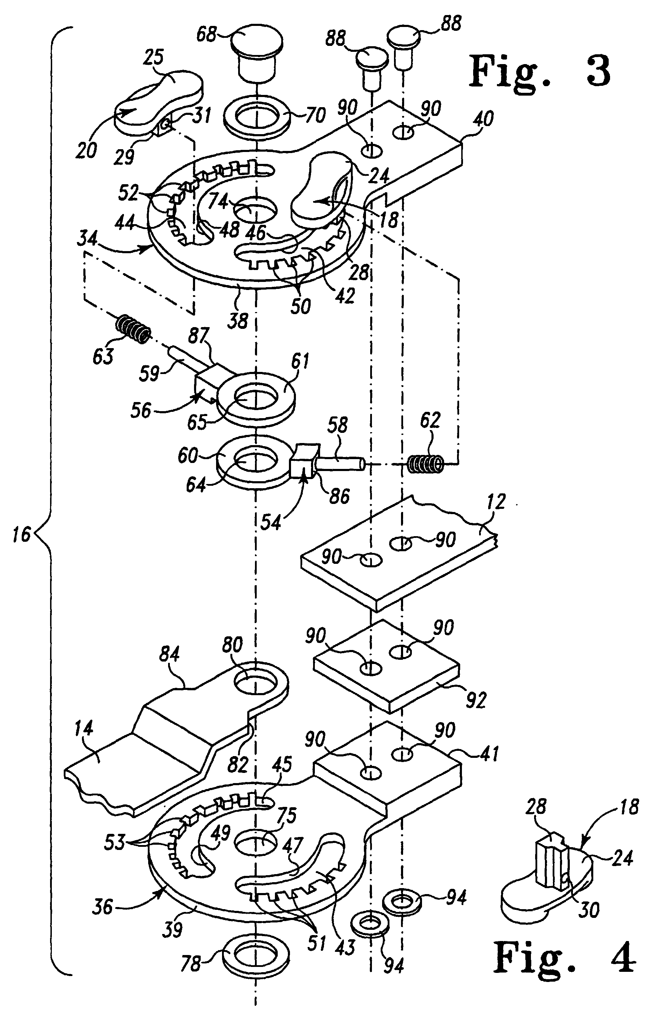

FIG. 3 is an exploded, perspective view of the first embodiment of the range of motion hinge assembly of FIGS. 2 and 2A showing the range of motion hinge assembly including a pair of opposing base plates, the opposing base plates each formed to include an arcuate-shaped extension slot with a plurality of circumferentially spaced notches adapted to receive an extension stop pin, an arcuate-shaped flexion slot with a plurality of circumferentially spaced notches adapted to receive a flexion stop pin and a centre aperture adapted to receive pivot rivet, the pivot rivet adapted to pivotally connect an upper and a lower washer and the opposing base plates, the opposing base plates sandwiching between them an extension pivot post, a flexion pivot post and the lower strut;
FIG. 4 is a perspective view of the underside of the extension stop pin from the first embodiment of the range of motion hinge assembly showing a mating aperture for receiving a head portion of the extension pivot post;
FIG. 5 is a cross-sectional view through the first embodiment of the range of motion hinge assembly, taken along line 5―5 of FIG. 2B, showing one of the stop pins biassed radially outward by a spring positioned between the pivot post and the stop pin to engage one of the plurality of notches, and the other of the stop pins in the disengaged position through the action of the depicted finger applying pressure radially inward against a second spring positioned between the other pivot post and the other stop pin;

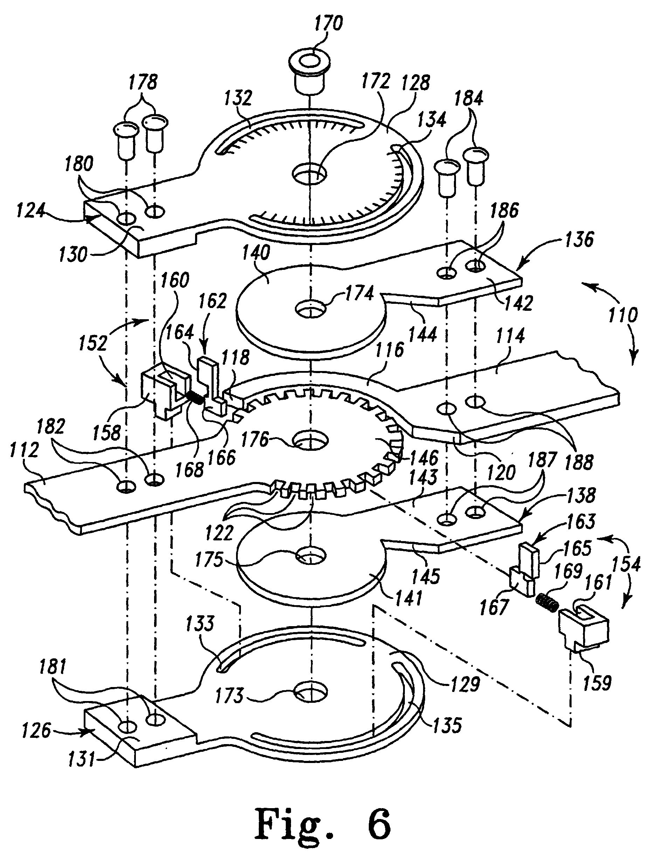

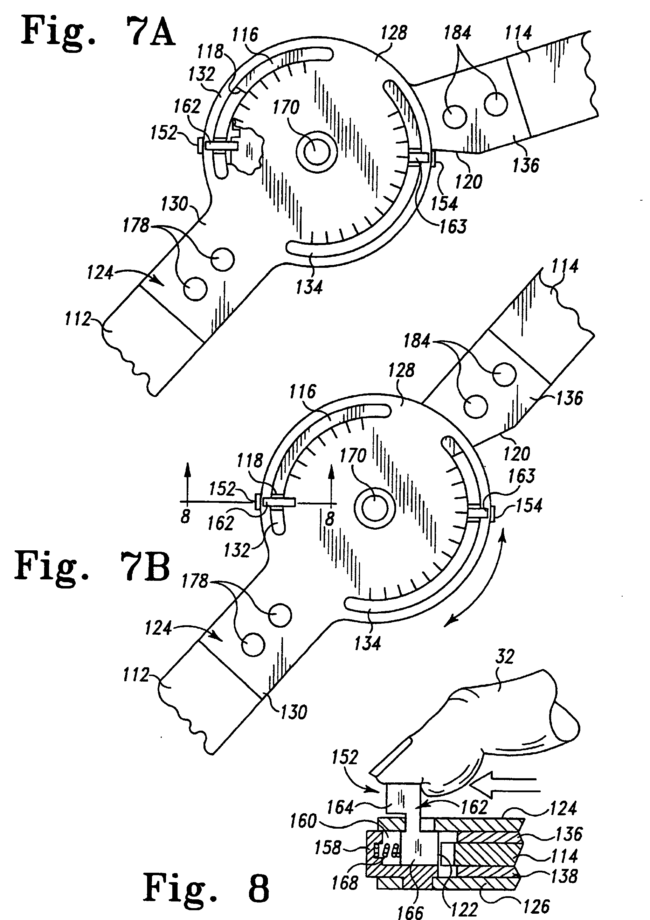

FIG. 6 is an exploded, perspective view of a second embodiment of the range of motion hinge of FIGS. 7A and 7B showing an outer upper and an opposing outer lower base plate rigidly connected to a lower strut, the opposing outer base plates each including an arcuate-shaped extension slot adapted to guide an extension stop pin and an arcuate-shaped flexion slot adapted to guide a flexion stop pin, an inner upper and an opposing inner lower base plate rigidly connected to a lower strut, the lower strut and the opposing inner base plates in combination forming a flexion-stop surface, and the lower strut having an arcuate-shaped extension that ends in an extension-stop surface, the central end of the upper strut having formed about its periphery a plurality of circumferentially spaced notches for receiving the extension and the flexion stop pins, and the upper strut being nested within the lower strut arcuate extension and being sandwiched between the two pairs of opposing base plates, the two pairs of opposing base plates and the upper strut pivotally connected by a pivot rivet that runs through an aperture formed in each of their respective centres, the extension stop pin and the flexion stop pin each having a stopping portion for engaging the notches on the upper strut and impinging the stop surfaces on the lower strut and each stop pin having a stop portion and a u-shaped compartment for receiving a z-shaped control piece, each z-shaped control piece having a manipulation portion and an engaging portion;
FIGS. 7A and 7B are side elevational views depicting the second embodiment of the range of motion hinge of FIG. 1, showing an arbitrary degree of flexion in FIG. 7A and a full degree of extension in FIG 7B;
FIG. 8 is a cross-sectional view through the second embodiment of the range of motion hinge assembly, taken along line 8―8 of FIG. 7B, showing one of the stop pins with a biassing spring disposed between the control piece and the stop-pin u-shaped compartment;
FIG. 9 is a perspective view of a third embodiment of a range of motion hinge;
FIG. 10 is an enlarged, partially exploded view of the third embodiment of the range of motion hinge of Fig. 9; and
FIG. 11 is an enlarged, completely exploded view of the third embodiment of the range of motion hinge of FIG. 9.

In the drawings, corresponding reference characters indicate corresponding parts throughout the several views.

Referring to the drawings, Fig. 1 shows an orthopaedic brace 10 which is adapted to fit on a human limb in order to limit the range of motion of a human joint. While it is expected that this improved orthopaedic brace is adaptable to any of the joints of the human body, it will be described herein as used to aid the healing of the knee joint. Consequently, Fig. 1 shows the orthopaedic brace operatively connected to a human leg 11 through conventional means. The orthopaedic brace 10 includes an upper strut 12, a lower strut 14 and an improved range of motion hinge assembly 16 disposed between the upper strut 12 and the lower strut 14. The upper strut 12 is operatively attached to the thigh portion of the leg 11 by an upper attachment/strap device 13, while the lower strut 14 is operatively attached to the calf portion of the leg 11 by a lower attachment/strap device 15. In this manner, the orthopaedic brace 10 is operatively attached to the user's leg 11. It should be appreciated that the orthopaedic brace 10 typically includes a hinge and an upper and lower strut assembly as depicted in Fig. 1 on both sides of the leg 11, that is a mirror image thereof.

Referring to Fig. 2A, the improved range of motion hinge assembly 16 is shown in an arbitrary degree of flexion, while Fig. 2B shows the improved range of motion hinge assembly 16 in a full degree of extension. The preferred embodiment contemplates a total included angle of, for example, about 140 degrees between full flexion and full extension. The extension angular limit and the flexion angular limit, respectively, are controlled by interaction between an extension stop pin assembly 18 and a plurality of extension stop notches 50 and 51 of arcuate extension slots 42 and 43, respectively, of the base plates 34 and 36, respectively, and interaction between a flexion stop pin assembly 20 and a plurality of flexion stop notches 52 and 53 of arcuate extension slots 44 and 45, respectively, of base plates 34 and 36, respectively (see Fig. 3).

Figs. 2A, 2B, 3 and 4 depict the improved range of motion hinge assembly 16, which pivotally interconnects the upper strut 12 and the lower strut 14. The hinge assembly 16 includes a pair of outer and inner opposing base plates 34 and 36, that are preferably identically configured. Each of the opposing base plates 34 and 36 has a generally circular body portion 38 and 39, respectively, from which generally rectangular connecting tab portions 40 and 41, respectively, tangentially extend. Formed through the body portions 38 and 39 are an aligned pair of arcuate extension slots 42 and 43 about the periphery of the body portions 38 and 39, respectively. Also formed along the periphery and through the body portions 38 and 39 are an aligned pair of arcuate flexion slots 44 and 45 circumferentially spaced from the aligned extension slots 42 and 43, respectively. The extension slots 42 and 43 have circumferentially unobstructed radially inner portions 46 and 47, respectively, and the flexion slots 44 and 45 likewise have circumferentially unobstructed radially inner portions 48 and 49, respectively.

The radially outer sides of the extension slots 42 and 43 are provided with seven radially outwardly extending notches 50 and 51, respectively, each of such notches being circumferentially spaced from its immediately adjacent notch or notches by an angle, for example, of approximating 15 degrees. As subsequently will be described, the pair of circumferentially lowermost notches of the plurality of pairs of notches 50 and 51 in the opposing base plates 34 and 36 correspond to an extension limit angle of 0 degrees (at which the leg 11 is fully straightened) as depicted in Fig. 2B. Each counterclockwise successive pair of notches 50 and 51 increases the extension limit angle by the amount of degrees between each notch (at which the leg 11 is less than fully straightened) as depicted in Fig. 2A. It should be appreciated that the [number of] incremental angles (i.e. notches) and the maximum angle of extension may vary.

Similarly, each of the radially outer sides of the aligned flexion slots 44 and 45 has formed therein ten radially outwardly extending notches 52 and 53, respectively, circumferentially spaced apart from one another by an angle of, for example, 10 to 15 degrees. The pair of circumferentially uppermost notches of the plurality of pairs of notches 52 and 53 in the opposing base plates 34 and 36 in Fig. 3 correspond as described below to a maximum flexion angle of, for example, 135 degrees, with each clockwise successive pair of notches 52 and 53 reducing such flexion angle by the number of degrees between the plurality pairs of notches 52 and 53 until, at the circumferentially lowest notches thereof, the flexion limit angle is 0 degrees (at which the leg 11 is fully straightened). It will be appreciated that the [number of] incremental angles (i.e. notches) and the maximum angle of flexion may vary.

The central end portion of the upper strut 12 is sandwiched and retained between the opposing base plates 34 and 36, as shown in Figs. 2A and 2B, and anchored to the connecting tabs 40 and 41 of the opposing base plates 34 and 36 by means of two rivets 88 which are extended down into aligned openings 90 (Fig. 3) formed through the tabs 40 and 41, through a spacer piece 92, through the upper strut 12, and through two washers 94. The central end portion of the lower strut 14 is also sandwiched and retained between the opposing base plates 34 and 36.

The central end portion of the lower strut 14 has formed through it a circular opening or aperture 80, which is aligned with central circular openings or apertures 74 and 75 formed through the respective circular body portions 38 and 39 of respective opposing base plates 34 and 36. Also sandwiched and retained between the body portions 38 and 39 of the respective opposing base plates 34 and 36 is an extension pivot post 54 and a flexion pivot post 56 (Fig. 3).

The pivot posts 54 and 56 each have a generally elongated shank portion 58 and 59, respectively, and a generally rounded head portion 60 and 61, respectively. The head portions 60 and 61 each have formed through their centres a circular aperture 64 and 65, respectively. A pivot rivet 68 pivotally connects, by sequentially extending down through a washer 70, the central circular opening 74 through the circular body portion 38 of the outer opposing base plate 34, the circular apertures 64 and 65 through the head portions 60 and 61 of the pivot posts 54 and 56, respectively, the circular opening 80 through the lower strut 14, the central circular opening 75 through the circular body portion 39 of the inner opposing base plate 36, and through a washer 78 (Fig. 3).

The central end of the lower strut 14 has formed thereon along its right edge (as viewed in Fig. 3) a generally concave, arcuately shaped extension stop surface 82. Opposite the extension stop surface 82, formed along the left edge of the central end of the lower strut 14 is a generally concave, arcuately shaped flexion stop surface 84.

The illustrated improved range of motion hinge assembly 16 is further provided with an extension stop-pin assembly 18 and a flexion stop-pin assembly 20. Referring to Figs. 3 and 4, the stop-pin assemblies 18 and 20 each have a manipulation portion 24 and 25, respectively, a stop portion 28 and 29, respectively, that extends axially inward from and tangential to the respective manipulation portions 24 and 25, and a mating aperture 30 and 31 formed radially through the stop portions 24 and 25, respectively. The mating apertures 30 and 31 are adapted to receive the respective shank portions 58 and 59 of the pivot posts 54 and 56, respectively, with a respective bias spring 62 and 63 disposed over the respective shank portions 58 and 59. The springs 62 and 63 rest or are seated against and disposed between a respective spring seat 86 and 87 of the pivot posts 54 and 56, respectively, and the surface surrounding each mating aperture 30 and 31.

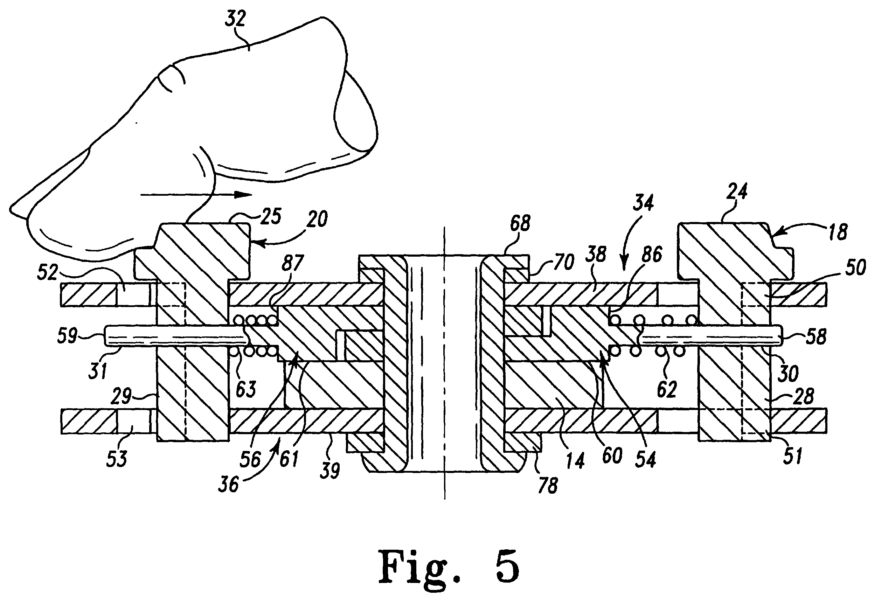

Fig. 5 depicts the cooperative operation of the pivot post assemblies 54 and 56 and the stop-pin assemblies 18 and 20. The pivot post assemblies 54 and 56 keep the stop-pin assemblies 18 and 20 nestled within the extension and flexion slot pairs 42 and 43, and 44 and 45, respectively. The bias spring 62 urges the extension stop-pin assembly 18 radially outwardly from the pivot rivet 68 until the stop portion 28 selectively engages one of the plurality of pairs of extension notches 50 and 51. The bias spring 62 locks the stop portion 28 into the selected extension notch pair 50 and 51. Although not depicted as such in Fig. 5, the flexion pivot-post assembly 56 and the flexion stop-pin assembly 20 are adapted to operate in the same manner to selectively engage and captively lock into one of the plurality of pairs of flexion notches 52 and 53. Also illustrated in Fig. 5 is how to disengage the stop-pin assemblies 18 and 20.

Fig. 5 shows a finger 32 applying radially inward pressure to the manipulation portion 25 of the flexion stop-pin assembly 20 thereby moving the entire stop-pin assembly 20 radially inwardly along the shank portion 59 of the flexion pivot post assembly 56, which in turn compresses the bias spring 63 and disengages the stop portion 29 of the flexion stop-pin assembly 20 from the selective pair of flexion stop notches 52 and 53. By continuing to apply radially inward pressure on the manipulation portion 25 of the flexion stop-pin assembly 20, the stop-pin assembly 20 may be pivotally translated circumferentially within the flexion slots 44 and 45 and about the axis formed by the flexion pivot post 56 and the pivot rivet 68 to selectively engage another one of the plurality of circumferentially spaced flexion notch pairs 52 and 53. Upon easing the pressure applied to the manipulation portion 25, the spring 63 urges the stop portion 29 to engage and lock into one of the plurality of pairs of flexion notches 52 and 53, as selected, as described above for the extension stop-pin assembly 18. The extension stop-pin assembly 18 is disengaged and translated in identical fashion to that just described for the flexion stop-pin assembly 20.

After the stop-pin assemblies 18 and 20 have been adjusted to captively engage the desired extension and flexion notches 50 and 51, respectively, and 52 and 53, respectively, in the manner previously described, the movement of the lower strut 14 with respect to the upper strut 12, one relative to the other, will be restricted to the desired range of motion as follows. The extension stop surface 82 formed on the central end of the lower strut 14 will contact and immovably impinge upon the captively engaged stop portion 28 of the extension stop-pin assembly 18, thereby defining the extension angular limit of the hinge assembly 16. Similarly, the flexion stop surface 84 formed on the opposite side of the central end of the lower strut 14 will contact and immovably impinge upon the captively engaged stop portion 29 of the flexion stop-pin assembly 20, thereby defining the flexion angular limit of the hinge assembly 16.

An alternate embodiment of orthopaedic brace 10 is shown in Figs. 6, 7A, 7B and 8. In the alternate embodiment, the hinge assembly 110, which pivotally connects an upper strut 112 and a lower strut 114 includes an outermost outer base plate 124 and an identically configured opposing innermost inner base plate 126. Each of these opposing base plates 124 and 126 has a generally circular body portion 128 and 129, respectively, from which a generally rectangular connecting tab portions 130 and 131, respectively, tangentially extend. Formed through the body portions 128 and 129 are an aligned pair of arcuate extension slots 132 and 133, respectively, situated about the periphery of the respective body portions 128 and 129. Also formed along the periphery and through the body portions 128 and 129 are an aligned pair of arcuate flexion slots 134 and 135, respectively, that are circumferentially spaced from the aligned extension slots 132 and 133. The circular ends 130 and 131 each have formed through their centres a respective aperture 172 and 173.

The hinge assembly 110 also includes an innermost outer base plate 136 and an identically configured opposing outermost inner base plate 138. Each of these opposing base plates 136 and 138 has a respective generally circular body portion 140 and 141 from which a respective generally rectangular connecting tab portion 142 and 143 tangentially extends. Each of the opposing base plates 136 and 138 has formed on its left side a respective flexion stop surface 144 and 145 that slants obliquely inward from the respective tab portion 142 and 143. Each of the opposing base plates 136 and 138 has formed through the centre of their respective circular body portions 140 and 141 a circular aperture 174 and 175.

The central end portion of upper strut 112 is sandwiched and retained between the opposing base plates 124 and 126 and anchored to the connecting tabs 130 and 131 of the respective opposing base plates 124 and 126 by means of two rivets 178 that extend down through aligned openings 180 and 181 formed through the tab portions 130 and down through the openings 182 formed through the upper strut 112. The central end portion of the strut 112 that extends past the openings 182 has a generally rounded end 146 with a plurality of circumferentially spaced notches 122 around its periphery, and a circular aperture 176 formed through its centre.

The central end portion of the lower strut 114 is sandwiched and retained between opposing base plates 136 and 138 and anchored to the connecting tab portions 142 and 143 of the respective opposing base plates 136 and 138 by means of two rivets 184 that extend down through aligned openings 186 formed through the tab portion 142, down through the openings 188 formed through the lower strut 114 and down through openings 187 formed through the tab portion 143. The central end of the lower strut 114 has formed on its left side a flexion stop surface 120 that slants obliquely inward and gives way to a generally arcuately shaped hook-like appendage 116. The appendage 116 has formed at its end an extension stop surface 118.

The two sets of opposing base plates 124 and 126, and 136 and 138, are pivotally connected by pivot rivet 170, which sequentially runs down through the aperture 172 on the base plate 124, the aperture 174 on the base plate 136, the aperture 176 on the upper strut 112, the aperture 175 on the base plate 138, and the aperture 173 on the base plate 126, thereby movably sandwiching between the opposing plates 124 and 126, an extension stop-pin assembly 152 and a flexion stop-pin assembly 154.

The stop-pin assemblies 152 and 154 include a respective stopping portion 158 and 159 with a respective u-shaped notch 160 and 161 formed thereon with a respective control portion 162 and 163 that fits in the u-shaped notch 160. The control portions 162 and 163 each has a respective manipulation end 164 and 165 that extends axially toward the base plate 124, and extends through the respective slots 132 and 134. The control portions 162 and 163 each also has a respective locking portion 166 and 167 that selectively engages the plurality of notches 122. Disposed between the control portion 162 and the u-shaped notch 160 is a bias spring 168. Disposed between the control portion 163 and the u-shaped notch 161 is a bias spring 169.

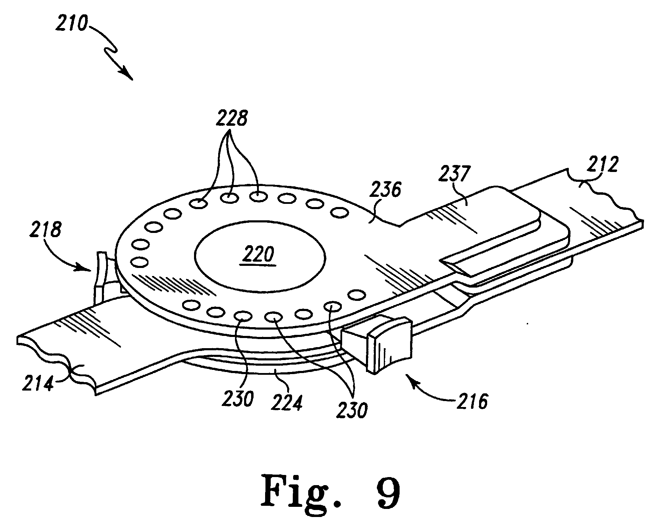

Referring additionally to Fig. 8, by applying radially outward pressure on the manipulation portion 164 of the control portion 162 of either stop-pin assemblies 152 or 154 (with the stop-pin assembly 152 shown and hereafter described with the understanding that the stop-pin assembly 154 functions in the same manner), the bias spring 168 is compressed allowing the stop portion 158 to disengage one of the plurality of notches 122 and thereby allowing the stop-pin assembly 154 to be translated clockwise or counterclockwise in the slot 132. By releasing the pressure on the manipulation portion 164, the bias spring 168 urges and captively holds the stop portion 158 into the selected notch 122. When the lower strut 114 is moved about the axis defined by pivot rivet 170 the extension-stop surface 118 will come into contact with the extension stop-pin assembly 152, thereby defining the extension angular limit. Similarly, when the lower strut 114 is moved in the opposite direction, the flexion-stop surfaces 120 and 144 will come into contact with the flexion stop-pin assembly 154, thereby defining the flexion angular limit (Figs. 6, 7A, 7B and 8).

Referring to Fig. 9, there is shown a third embodiment of a range of motion (ROM) hinge assembly, generally designated 210, in accordance with the principles of the present invention. The ROM hinge assembly 210 pivotally couples an upper strut 212 with a lower strut 214. The ROM hinge assembly 210 includes an extension stop assembly 216 for selectively setting an extension range of motion, and a flexion stop assembly 218 for selectively setting a flexion range of motion. Both the extension stop assembly 216 and the flexion stop assembly 218 are radially actuated in like manner to the respective stop assemblies of the other two embodiments described herein, such that they are normally radially biassed into a disengagably or releasably locked position.

The extension stop assembly 216 can be selectively positioned in one of a plurality of settings each of which defines a particular range of motion for the extension of a joint of a user (i.e. wearer of the orthopaedic brace 10). The ROM hinge assembly 210 has seven extension settings or positions. The user can identify the extension setting that the extension stop assembly 216 is in by extension position apertures 230 in an upper cover plate 236 and extension position apertures 298 in a lower cover plate 296. In like manner, the flexion stop assembly 218 can be selectively positioned in one of a plurality of settings, each of which defines a particular range of motion for the flexion of a joint of a user (i.e. wearer of the orthopaedic brace 10). The ROM hinge assembly 210 has ten flexion settings or positions. The user can identify the flexion setting that the flexion stop assembly 218 is in by flexion position apertures 228 in the cover plate 236 and flexion position apertures 299 in the lower cover plate 296. The flexion stop assembly 218 acts and can be set independent of the extension stop assembly 216. It should be appreciated that the number of settings and/or positions of both the extension and flexion ranges of motion may vary. As well, the extension and flexion positions herein define increments of movement of approximately 15 degrees, but other increments may be used.

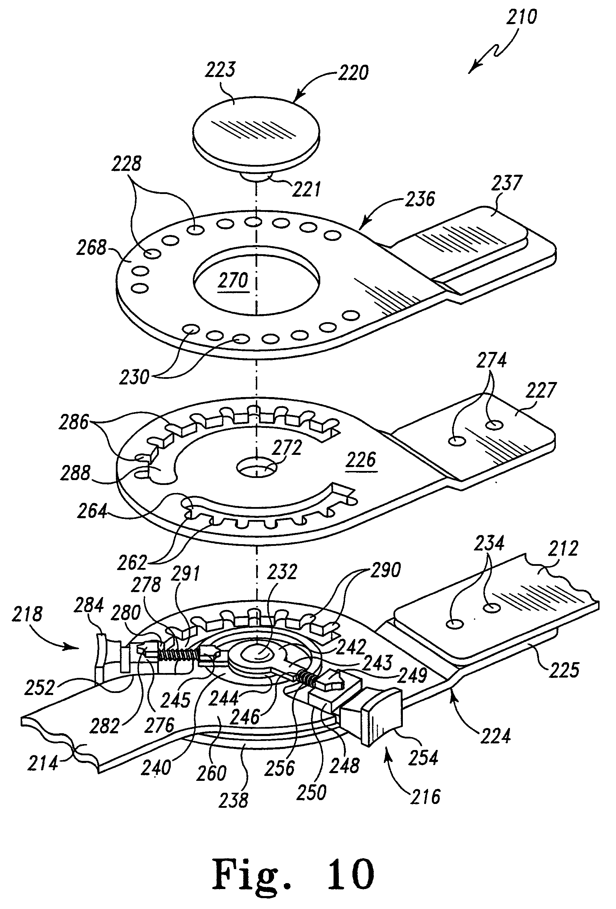

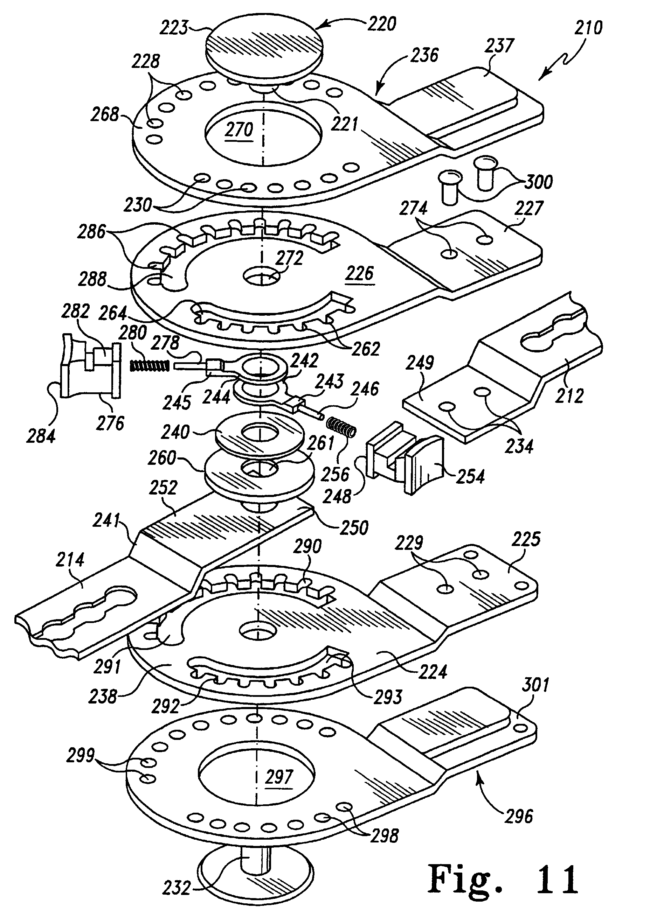

With reference to Figs. 10 and 11, the various components of the ROM hinge assembly 210 is shown in an exploded view. The upper strut 212 is coupled to an end 225 of a lower base plate 224 via fasteners (e.g. rivets) 300 that extend through openings 234 in an end portion of the upper strut 212 and openings 229 in the end 225 of the lower base plate 224. The fasteners 300 also couple an end 227 of the upper base plate 226 by extending down through openings 274. The end 237 of the upper cover 236 is fashioned to cover the heads of the fasteners 300. An end 301 of the lower cover 296 is fashioned to cover the ends of the fasteners 300. The lower strut 214 includes a rounded end 260 that is pivotally coupled to a circular end 238 of the lower base plate 224 via pivot ferrule 232 that extends through an opening 261 in the end 260, an upper washer 240 and a lower washer 241. The pivot ferrule 232 provides a pivot point for an extension eyelet 242 and a flexion eyelet 244. The pivot ferrule 232 also provides a coupling point for a flange 221 of the pivot pin 220 which extends through an opening 270 in the body 268 of the upper cover 236, an opening 272 in the upper base plate 226, and into the pivot ferrule 232. The pivot pin 220 sandwiches and retains the hinge assembly 210 together. In addition, the pivot pin 220 includes a cap portion 223 that is retained within the opening 270 of the upper base plate 236.

The pivot ferrule 232 also extends through an opening 297 in the lower cover plat 296 and pivotally carries an extension eyelet 242 having an extension arm 243, and a flexion eyelet 244 having a flexion arm 245. The extension eyelet 242 and extension arm 243 cooperate with the extension stop assembly 216 to set the extension range of motion, while the flexion eyelet 244 and flexion arm 245 cooperate with the flexion stop assembly 218 to set the flexion range of motion in the following manner.

The extension arm 243 includes an aperture into which an extension shaft 246 is slidably retained. An extension biassing spring 256 surrounds the extension shaft 246 and is retained between the extension arm 243 and an extension head 248 of the extension stop assembly 216. The extension head 248 radially outwardly terminates in a manipulation surface 254. The extension biassing spring 256 normally biases the extension stop assembly 216 radially outwardly, such that an upper extension head stop flange 249 engages a selective one of a plurality of extension notches 262 along the outer circumferential periphery of an upper extension slot 264 formed in the upper base plate 226. The arcuate length of the upper extension slot 264 defines the total extension range of movement, while the number and distance between the plurality of notches 262 sets the actual extension range of movement. The lower base plate 224 has a like extension slot 293 with a plurality of extension notches 292 that movably retain a lower extension head stop flange (not seen) formed on the lower surface of the extension head 248. The extension stop assembly 216 is thus configured to arcuately move within the extension slot pair to engage any one of the selective pair of notches to set the extension range of motion.

The extension stop assembly 216 is set by disengaging the extension head 248 from an extension notch pair by radially inward pressure, moving the stop assembly, and releasing pressure to allow the normal bias of the spring 256 to radially outwardly urge the head 248 into engagement with an extension notch pair. The end 260 of the lower strut 214 includes an extension stop surface 250 that limits the extension range of movement of the lower strut 214, as set by the extension stop assembly 216, by providing an abutment between the extension stop surface 250 and the extension head 248.

The flexion arm 245 includes an aperture into which a flexion shaft 278 is slidably retained. A flexion biassing spring 280 surrounds the flexion shaft 278 and is retained between the flexion arm 245 and a flexion head 276 of the flexion stop assembly 218. The flexion head 276 radially outwardly terminates in a manipulation surface 284. The flexion biassing spring 280 normally biases the flexion stop assembly 218 radially outwardly, such that an upper flexion head stop flange 282 engages a selective one of a plurality of flexion notches 286 along the outer circumferential periphery of an upper flexion slot 290 formed in the upper base plate 226. The arcuate length of the upper flexion slot 290 defines the total flexion range of movement, while the number and distance between the plurality of notches 288 sets the actual flexion range of movement. The lower base plate 224 has a like flexion slot 291 with a plurality of flexion notches 290 that movably retain a lower flexion head stop flange (not seen) formed on the lower surface of the flexion head 276. The flexion stop assembly 218 is thus configured to arcuately move within the flexion slot pair to engage any one of the selective pair of notches to set the flexion range of motion.

The flexion stop assembly 218 is set by disengaging the flexion head 276 from a flexion notch pair by radially inward pressure, moving the stop assembly, and releasing pressure to allow the normal bias of the spring 280 to radially outwardly urge the head 276 into engagement with a flexion notch pair. The end 260 of the lower strut 214 includes a flexion stop surface 252 that limits the flexion range of movement of the lower strut 214, as set by the flexion stop assembly 218, by providing an abutment between the flexion stop surface 252 and the flexion head 276.

The manipulation portions or buttons 254 and 284 of the respective extension stop assembly 216 and the flexion stop assembly 218 do not extend axially upwardly through the base plates as in the other embodiments. Rather, the buttons 254 and 284 radially extend from the hinge assembly 210 to create an even lower profile hinge assembly.

## Claims

1. An orthopaedic brace comprising:
a first strut;
a second strut; and
a hinge assembly disposed between the first strut and the second strut and configured to provide movement of one of the first and second struts about a pivot axis, the hinge assembly including a radially actuated stop assembly configured to restrict the movement of the one of the first and second struts to a selected range of motion.

2. A brace as claimed in claim 1, in which the radially actuated stop assembly is configured to restrict the movement of the one of the first and second struts to a selected range of motion with respect to flexion of a joint of a user which corresponds to the pivot axis.

3. A brace as claimed in claim 2, in which the hinge assembly further comprises:
a second radially actuated stop assembly configured to restrict the movement of the one of the first and second struts to a selected range of motion with respect to extension of the joint of the user which corresponds to the pivot axis.

4. A brace as claimed in claim 3, in which the first and second stop assemblies are each releasably, radially biassed into a selected, respective range of motion.

5. A brace as claimed in claim 1, in which the radially actuated stop assembly is configured to restrict the movement of the one of the first and second struts to a selected range of motion with respect to extension of a joint of a user which corresponds to the pivot axis.

6. An orthopaedic brace comprising:
an upper strut;
a lower strut;
a radially actuated stop assembly; and
a hinge assembly disposed between the upper strut and the lower strut and configured to provide movement of one of the upper and lower struts about a pivot axis corresponding to a joint axis of a user, the hinge assembly including a plurality of notches corresponding to a plurality of ranges of motion and configured to coact with the radially actuated stop assembly to restrict the movement of the one of the upper and lower struts to a selective one of the plurality of ranges of motion.

7. A brace as claimed in claim 6, in which the plurality of ranges of motion are with respect to extension of the joint of the user.

8. A brace as claimed in claim 6, in which the plurality of ranges of motion are with respect to flexion of the joint of the user.

9. A brace as claimed in claim 8, further comprising:
a second radially actuated stop assembly; and
the hinge assembly including a second plurality of notches corresponding to a second plurality of ranges of motion and configured to coact with the second radially actuated stop assembly to restrict the movement of the one of the upper and lower struts with respect to extension of the joint of the user.

10. A brace as claimed in claim 9, in which the first stop assembly is releasably, radially biassed into a selected one of the plurality of ranges of motion, and the second stop assembly is releasably, radially biassed into a selected one of the second plurality of ranges of motion.

11. A brace as claimed in claim 10, in which the first stop assembly is releasably, radially outwardly biassed by a first spring, and the second stop assembly is releasably, radially outwardly biassed by a second spring.

12. A brace as claimed in claim 9, in which the lower strut includes:
a flexion stop surface configured to impinge the first radially actuated stop assembly; and
an extension stop surface configured to impinge the second radially actuated stop assembly.

13. A brace as claimed in claim 9, in which the hinge assembly further includes a first base plate and a second base plate, and the first plurality of notches extend from a first pair of aligned slots formed in the first and second base plates, and the second plurality of notches extend from a second pair of aligned slots formed in the first and second base plates.

14. An orthopaedic brace comprising:
an upper strut;
a lower strut;
a radially actuated extension stop assembly;
a radially actuated flexion stop assembly; and
a hinge assembly disposed between the upper and lower struts and configured for movement of one of the upper and lower struts about a pivot axis corresponding to a joint axis of a user, the hinge assembly including a pair of opposing base plates coupled to one of the upper and lower struts and pivotally retaining the other of the upper and lower struts between them, the pair of opposing base plates each having an extension slot with a plurality of extension notches and a flexion slot with a plurality of flexion notches, the extension slots retaining the radially actuated extension stop assembly for translation therein to selectively engage one of the plurality of pairs of extension notches to restrict movement of the upper and lower struts to a selected range of extension motion, and the flexion slots retaining the radially actuated flexion stop assembly for translation therein to selectively engage one of the plurality of pairs of flexion notches to restrict movement of the upper and lower struts to a selected range of flexion motion.

15. A brace as claimed in claim 14, in which the lower strut includes:
a flexion stop surface configured to impinge the radially actuated flexion stop assembly; and
an extension stop surface configured to impinge the radially actuated extension stop assembly.

16. A brace as claimed in claim 14, in which the radially actuated extension stop assembly is releasably biassed into a selected one of the plurality of pairs of extension notches, and the radially actuated flexion stop assembly is releasably biassed into a selected one of the plurality of pairs of flexion notches.

17. A brace as claimed in claim 16, in which the radially actuated extension stop assembly is biassed by an extension spring, and the radially actuates flexion stop assembly is biassed by a flexion spring.

18. A brace as claimed in claim 14, in which the extension slots of the pair of opposing base plates are arcuate-shaped and the plurality of extension notches extend radially from a periphery thereof, and the flexion slots of the pair of opposing base plates are arcuate-shaped and the plurality of flexion notches extend radially from a periphery thereof.

19. A brace as claimed in claim 14, in which the plurality of flexion notches and the plurality of extension notches are circumferentially spaced at fifteen degree increments.

20. A brace as claimed in claim 14, in which the plurality of flexion notches is greater than the plurality of extension notches.
